# EUROPÄISCHE PATENTSCHRIFT

(11) **EP 1 841 853 B1**
(45) Veröffentlichungstag und Bekanntmachung des Hinweises auf die Patenterteilung: **23.04.2008**
(21) Anmeldenummer: 05798733.1
(22) Anmeldetag: 04.11.2005
(51) Int. Cl.: C12M 1/113, C12P 5/02, B01F 7/04

(54) **VERFAHREN UND VORRICHTUNG ZUM BETRIEB EINES LIEGENDEN PFROPFSTROMBETRIEBENEN FERMENTERS**
METHOD AND DEVICE FOR OPERATING A HORIZONTAL PLUG-FLOW FERMENTER
PROCEDE ET DISPOSITIF POUR FAIRE FONCTIONNER UN FERMENTEUR HORIZONTAL COMMANDE PAR UN FLUX GREFFE

(30) Priorität: 26.01.2005 CH 123052005
(43) Veröffentlichungstag der Anmeldung: 10.10.2007
(73) Patentinhaber: Kompogas AG, 8152 Glattbrugg (CH)
(72) Erfinder: SCHMID, Walter, CH-8152 Glattbrugg (CH)
(74) Vertreter: Schneider Feldmann AG Patent- und Markenanwälte
(86) Internationale Anmeldenummer: PCT/CH2005/000647
(87) Internationale Veröffentlichungsnummer: WO 2006/079228

(56) Entgegenhaltungen:
- EP-A- 0 324 894
- EP-A- 0 621 336
- EP-A- 0 770 675
- EP-A- 1 332 805
- DE-A1- 3 149 344
- DE-A1- 19 624 268
- DE-A1- 19 648 875
- US-A- 3 367 126
- US-A- 4 514 297
- US-A- 5 521 092
- DATABASE WPI Section Ch, Week 200332 Derwent Publications Ltd., London, GB; Class D15, AN 2003-336020 XP002361736 & JP 2002 308685 A (SUMITOMO HEAVY IND LTD) 23. Oktober 2002 (2002-10-23)
- DATABASE WPI Section Ch, Week 199339 Derwent Publications Ltd., London, GB; Class D16, AN 1993-309046 XP002361737 & JP 05 221764 A (NIPPON STEEL CORP) 31. August 1993 (1993-08-31)

## Beschreibung

Die vorliegende Erfindung betrifft ein Verfahren und eine Vorrichtung zum Betrieb eines pfropfströmbetriebenen, liegenden Fermenters zur anaeroben Fermentation biogener Abfälle mit einem Einlass und einem Auslass und einem Rührwerk, welches aus einer den Fermenter in seiner Längsrichtung durchquerenden Welle besteht, an der eine Vielzahl von Rührarmen angeordnet sind, wobei die Welle im Bereich der Fermenterenden gelagert ist.

Die Anmelderin ist die weltweit führende Anbieterin von Anlagen zur Erzeugung von Biogas aus biogenen Abfällen. Der prinzipielle Aufbau solcher Anlagen geht aus der europäischen Patentschrift EP-0'476'217-A hervor. Die Anlagen arbeiten nach einem Verfahren gemäss dem europäischen Patent EP-B-621'336. Das erstgenannte Schutzrecht offenbart einen pfropfstrombetriebenen liegenden Fermenter, der zur anaeroben Fermentation biogener Abfälle geeignet ist. Der Fermenter ist ein langgestreckter, liegender Tank mit einem an einem Ende vorgesehenen Einlass und einem am gegenüber liegenden Ende vorhandenen Auslass. Die biogenen Abfälle werden einlassseitig zerkleinert eingegeben und mit fermentiertem Gut und/oder Presswasser aus der Aufbereitung geimpft. Hierdurch wird das zu fermentierende Gut mit Methanbakterien angereichert. Im Fermenter werden nun unter kontrollierter Durchmischung die biogenen Abfälle unter Bildung von Biogas abgebaut und anschliessend nach dem Verlassen durch den Auslass einer aeroben Verrottung zugeführt.

Die weltweite Nachfrage nach Anlagen der eingangs genannten Art mit immer grösseren Kapazitäten führt dazu, dass auch immer grössere Fermenter gebaut werden. Um dies zu ermöglichen, müssen die Fermentertanks vor Ort errichtet werden, wobei dies entweder durch die segmentweise Zusammenfügung zu einem Stahltank erfolgen kann oder, wie aus der EP-770'675-A bekannt ist, kann der liegende Fermentertank vor Ort aus Beton erstellt werden. Zur Erhöhung der Kapazität werden heute liegende Fermentertanks mit einer Gesamtlänge von über 50 Metern und einem Durchmesser von über 10 Metern realisiert. Das Rührwerk muss nicht nur die biogenen Abfälle durchmischen um eine gewisse Homogenität zu erreichen, sondern gleichzeitig muss sichergestellt werden, dass schwere Festgüter, wie insbesondere Sand und Steine nicht am Boden des Fermentertanks sedimentieren und folglich nicht mehr ausgetragen werden. Obwohl der Fermenter im Pfropfstrom betrieben wird, vermag die Durchströmung die absinkenden Schwerstoffe nicht auszutragen, da die Pfropfstrombewegung nur eine geringe Strömungsgeschwindigkeit aufweist. Die Durchsatzzeit der biogenen Abfälle durch den Fermenter vom Einlass zum Auslass beträgt nämlich mehrere Tage. Das Rührwerk trägt folglich neben der Durchmischung ebenfalls dazu bei, diese Schwerstoffe vom Boden wieder nach oben zu befördern, um danach bei der nachfolgenden Sinkbewegung im Pfropfstrom Richtung Fermenterauslass transportiert zu werden. Entsprechend besteht das Rührwerk aus einer den Fermenter durchsetzenden Welle mit einer Vielzahl von Rührarmen, die an ihrem von der Welle abgelegenen Ende mit entsprechenden Schaufeln versehen sind.

Je grösser der Durchmesser des liegenden Fermenters ist, umso grösser ist das darin aufgenommene Volumen an biogenen Abfällen und umso problematischer wird das Problem der Sedimentation der schweren Feststoffe. Bei gleichbleibendem prozentualem Anteil an absinkenden schweren Feststoffen nimmt folglich die absolute Menge dieser Feststoffe zu. Durch das Rührwerk wird, wie bereits erwähnt, nicht nur die Durchmischung der Methanbakterien sichergestellt, sondern gleichzeitig arbeitet das Rührwerk die sedimentierten Anteile im Fermenter wieder in die oberen Schichten. Entsprechend werden diese absinkenden Feststoffe durch den liegenden Fermenter in einer Art Wellenbewegung durch den Fermenter hindurch transportiert.

Um diese Feststoffe im liegenden Fermenter von unten nach oben zu fördern, weisen wie erwähnt die Rührarme entsprechende Schaufeln auf. Diese liegen jedoch nicht direkt an der Mantelwand des Fermenters an, sondern kommen lediglich in einem Bereich nahe dieser Mantelwand vorbei. Je kleiner die absinkenden Feststoffe sind, umso wahrscheinlicher ist deren Sedimentierung auf dem Fermenterboden. Entsprechend wird sich nach einer gewissen Zeit trotz des Rührvorganges eine Sedimentschicht bilden. Die aufzuwendende Kraft des Rührwerkes nimmt entsprechend zu, umso stärker die Sedimentation fortgeschritten ist und umso länger die Stillstandszeiten sind. Die sedimentierte Schicht neigt zudem zu einer Verhärtung, und entsprechend müssten liegende Fermenter der hier interessierenden Art, die immer mit einem hohen Trockensubstanzanteil gefahren werden, nach längerer Betriebszeit entleert und entsprechend gereinigt werden.

Ein weiteres Problem, welches praktisch nur bei der Reinigung geleerter Fermenter festgestellt worden ist, besteht darin, dass durch die pfropfstrombetriebene Weise des Fermenters an den Rührarmen in Drehrichtung vorne gelegen sich immer mehr verfestigte Anteile der biogenen Abfälle aufbauen, wodurch insgesamt das Gewicht der Welle zunimmt. Diese Gewichtszunahme führt automatisch zu einer höheren Belastung der Welle des Rührwerkes und damit zu deren Durchbiegen. Dies wiederum führt dann dazu, dass die Schaufeln am Ende der Rührarme an der Fermenterwand reiben, wodurch das Drehmoment an der Welle nochmals steigt und zudem Abnutzungen an der Fermenterwand auftreten. All dies hat zu Schäden geführt, die oftmals erst Jahre nach der Inbetriebnahme auftauchten und deren Gründe anfänglich nicht erklärbar schienen.

Trotz verschiedener konstruktiver Massnahmen, um diesen Problemen zu begegnen, wurde hierfür keine sinnvolle Lösung gefunden. So hat man an der Welle Versteifungskonstruktionen angebracht oder die Rührarme des Fermenters mit Verkleidungen versehen.

Ein weiteres Problem, welches man insbesondere bei Revisionsarbeiten festgestellt hat, besteht darin, dass trotz vorgängiger Zerkleinerung des Grüngutes immer wieder längliche Feststoffe wie Seile, Drähte oder auch lange schlingenartige Grüngutabfälle in den Fermenter gelangen und sich während des Betriebes um die Welle herum aufwickeln.

Es ist folglich die Aufgabe der vorliegenden Erfindung, eine Lösung zu schaffen, welche die vorgenannten Probleme löst.

Die entsprechende Lösung wurde durch den erfindungsgemässen Betrieb gemäss Patentansprüchen erreicht.

Zur Ausübung des Verfahrens ist es erforderlich, dass man an den Enden der Rührarme besonders gestaltete Schaufeln aus zwei V-förmig angeordneten Scharblechen (15) angebracht hat, wie diese im Patentanspruch 1 und 7 beschrieben sind, als besonders vorteilhaft haben sich Schaufeln, wie diese im Patentanspruch 8 beschrieben sind, erwiesen.

In der anliegenden Zeichnung ist ein Ausführungsbeispiel des erfindungsgemässen Fermenters dargestellt und dessen Betrieb und Ausgestaltung mit Hinweis auf die anliegenden Zeichnungen erläutert. Es zeigt:
- Figur 1: einen vertikalen Längsschnitt durch einen Fermenter wie er erfindungsgemäss betrieben wird und
- Figur 2: einen vertikalen Schnitt durch den Fermenter quer zur Wellenlängsrichtung.
- Figur 3: zeigt eine Ansicht eines erfindungsgemäss ausgestalteten Rührarmes in der Ansicht senkrecht zur Wellenlängsachse und
- Figur 4: denselben Rührarm in einer Ansicht um 90° gedreht parallel zur Längsachse der Welle;
- Figur 5: zeigt einen Schnitt durch einen Rührarm entlang der Linie A-A gemäss Figur 4.

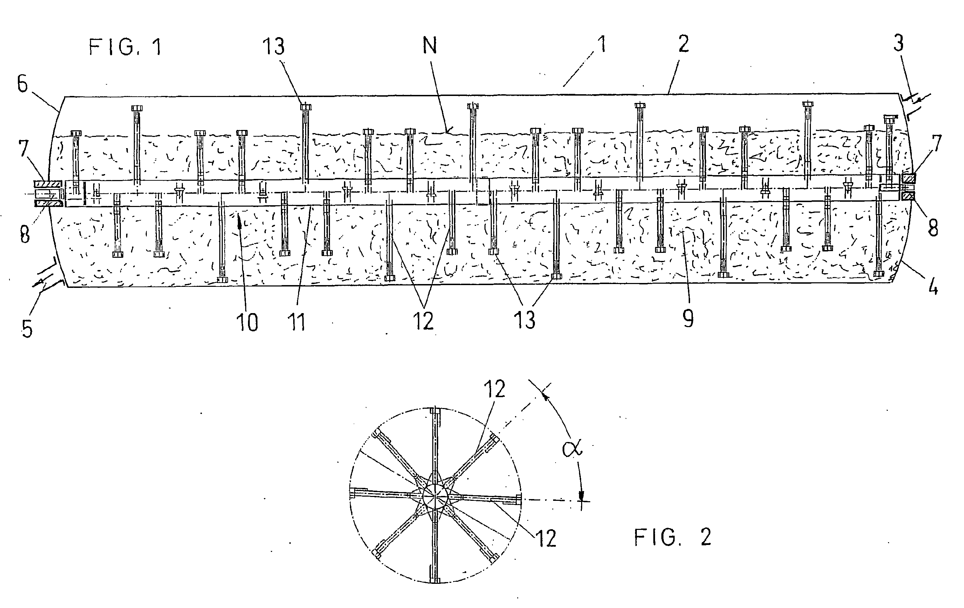

In der Figur 1 ist ein liegender Fermenter der hier interessierenden Bauart in seiner Gesamtheit in einem vertikalen Längsschnitt dargestellt. Der Fermenter ist mit 1 bezeichnet. Dieser umfasst den Fermentertank 2, der aus Stahl oder Beton gefertigt sein kann. Auf der einen Seite ist ein Einlass 3 in der einlassseitigen Stirnwand 4 vorhanden. Auf der gegenüber liegenden Seite ist ein Auslass 5 in der auslassseitigen Stirnwand 6 vorhanden. In den beiden Stirnwänden 4 und 6 ist je ein Wellenlager 7 angeformt, in dem die Welle 10 mit ihren endständigen Wellenzapfen 8 lagert.

Die Welle 10 umfasst die beiden Wellenzapfen 8, die drehfest mit einem Wellenkörper 11 verbunden sind. Der Wellenkörper 11 besteht aus einem Stahlrohr, welches beidseitig hermetisch verschlossen ist. Am Wellenkörper 11 sind eine Vielzahl von Rührarmen 12 mittels einer entsprechenden Schweisskonstruktion angebracht. Jeder Rührarm 12 weist endständige Schaufeln 13 auf.

Der Fermentertank 2 ist bis auf ein Niveau N mit zu vergärenden biogenen Abfällen 9 gefüllt. Da die Fermenter 1 beziehungsweise die Fermentertanks 2 aus ökonomischen Gründen immer grösser gebaut werden, bildet die unter ihrem Eigengewicht auftretende Durchbiegung der Welle ein Problem. Dieses Problem hat die Anmelderin gelöst, indem sie die Welle 10 als Hohlwelle gestaltet hat und so dimensioniert, dass der wirkende Auftrieb bei gefülltem Fermenter die mögliche Durchbiegung vollständig oder mindestens annähernd vollständig kompensiert.

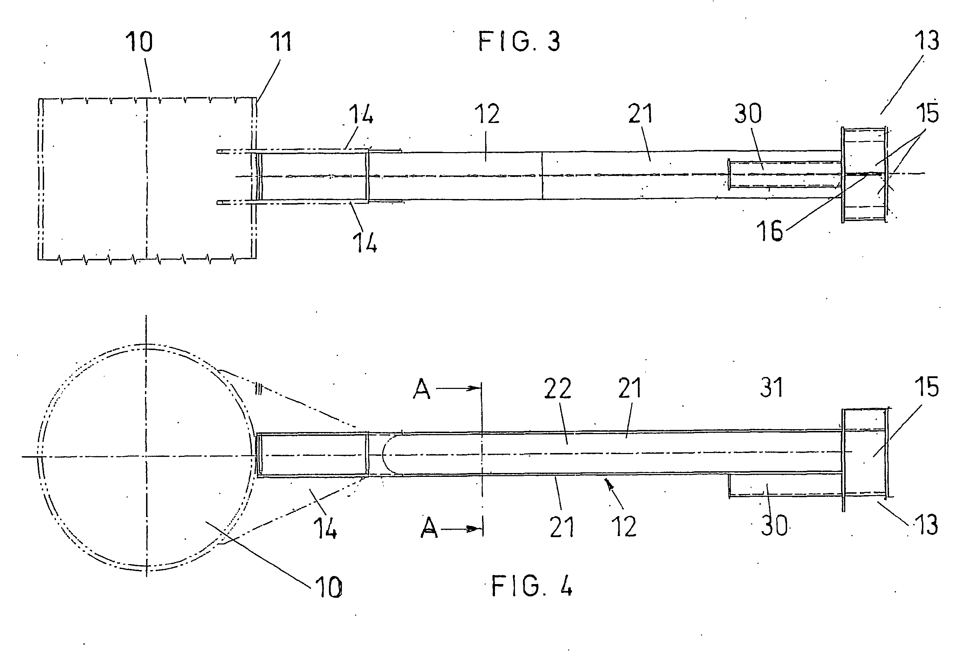

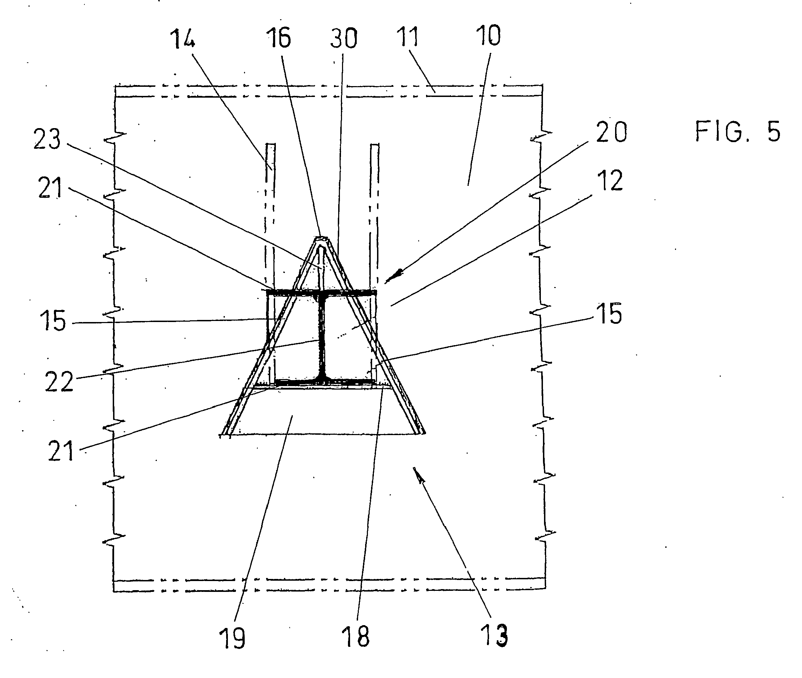

Die Grösse des Fermenters und damit auch die grosse Vielzahl an Rührarmen 12 mit entsprechenden Schaufeln 13 führt dazu, dass das anzulegende Drehmoment sehr hoch ist. Dieses anzulegende Drehmoment ist immer am grösstem beim Anlaufen der Welle 10. Nachdem eine Ruhephase im Fermenter geherrscht hat, sind entsprechend schwere Festkörperanteile auf dem Boden des Fermenters 1 sedimentiert. Um trotzdem den Fermenter vernünftig betreiben zu können, wird dieser mit Rührarmen 12 versehen, die besonders gestaltete Schaufeln 13 aufweisen. Diesbezüglich wird auf die nachfolgende Beschreibung der Figuren 3-5 verwiesen.

Die Rührarme 12 sind aus Doppel-T-Trägern 20 gestaltet. Ein solcher Doppel-T-Träger 20 weist zwei parallele Längsflanschen 21, sowie einen Verbindungssteg 22 auf. Der Doppel-T-Träger ist mittels Flanschplatten 14 auf den Wellenkörper 11 der Welle 10 aufgeschweisst. Sinnvollerweise verlaufen die Längsflanschen 21 parallel zur Längsachse der Welle 10. Bekanntlich ist die Rotationsgeschwindigkeit der Rührarme 12 im Fermenter wesentlich höher als die Pfropfstrombewegung, mit welcher der biogene Abfall 9 durch den Fermenten im Pfropfstrombetrieb durchgeschoben wird. Entsprechend decken die Längsflanschen 21 den vom Verbindungssteg 22 definierten Zwischenraum gegenüber den biogenen Abfällen 9 ab und verhindern ein Eintragen des Gutes in diesen Zwischenräumen unter dem Druck der Relativbewegung. Selbstverständlich kommen aber die biogenen Abfälle auch in diesen Bereich hinein, doch wird kein zusätzlicher Druck aufgebaut. Bei der Umkehrung der Drehbewegung wird zudem eine Abschälwirkung erzielt. Ferner decken die beidseits angeordneten Flanschplatten 14 den Zwischenraum im Bereich nahe der Welle ab.

Am Ende der Rührarme 12 sind die Schaufeln 13 angeordnet. Diese Schaufeln werden gebildet durch zwei in einem spitzen Winkel zueinander verlaufende Scharbleche 15, die eine gemeinsame Kante bilden und beispielsweise zusätzlich durch eine im Querschnitt V-förmige Schiene 16 versteift sein können. Vorzugsweise bildet der Verbindungssteg 22 des Doppel-T-Trägers 20 die Winkelhalbierende der beiden Scharbleche 15. Konstruktiv sind die Schaufeln so angeordnet, dass der Längsflansch 21, der näher bei der gemeinsamen Kante 16 liegt, im Bereich der Schaufel 13 entfernt ist. In diesem Bereich wird jedoch der Verbindungssteg 22 in Richtung der gemeinsamen Kante 16 der beiden Scharbleche 15 verlängert und bildet eine Versteifung der Konstruktion. Die Verlängerung ist hier mit 23 bezeichnet. Der zweite Längsflansch 21, welcher von der gemeinsamen Kante 16 weiter entfernt ist, dient als Stütze für das Schaufelblech 18. Am Ende des Rührarmes 12 beziehungsweise am Ende des Doppel-T-Trägers 20 ist senkrecht zu dessen Verlaufsrichtung ein Kratzblech 19 aufgeschweisst, welches zusammen mit den beiden Scharblechen 15 und dem Schaufelblech 18 den Aufnahmeraum der Schaufeln 13 bildet.

Zur reinen Förderung ebenso wie zum reinen Pflügen allein genügt die Ausgestaltung in der angegebenen Form. Da dabei bei grossen Fermentern prinzipiell auch das Ziel besteht, das Drehmoment zu reduzieren, ist es von Vorteil, die Scharbleche 15 im Bereich der Rührarme 12 über den Bereich der Schaufeln hinweg in Richtung zur Welle 10 hin zu verlängern. Diese Bugverlängerung 30 kann aus den Scharblechen 15 angeformt gebildet sein. Es ist aber auch möglich, die Bugverlängerung durch ein V-förmiges Bugblech 31 zu gestalten.

Prinzipiell ist es selbstverständlich denkbar, dass die Schaufeln beziehungsweise Pflugscharbleche anders ausgestaltet sind. Die hier angegebene Konstruktion ist jedoch eine Konstruktion, die unter Berücksichtigung der enormen Grössen auch entsprechend stark ausgebildet werden kann. Insbesondere ist noch darauf hingewiesen, dass die auftretenden Verschleisskräfte durch Abreibung relativ gross sind. Entsprechend kann man die gesamten Schaufeln aus entsprechend gehärteten Materialien herstellen. Angesichts der Dimensionen ist es jedoch nicht möglich, die gesamten Schaufeln aus speziellem Hartmetall zu fertigen. Die Kosten solcher Schaufeln wären immens. Entsprechend versieht man lediglich die Kanten der Schaufeln mit entsprechend gehärteten Schienen. Solche Schienen können, wie bereits erwähnt, an der gemeinsamen Kante 16 angeordnet sein, aber auch die Vorderkante des Kratzbleches sowie die von der gemeinsamen Kante 16 abgelegenen Endkanten der Scharbleche können mit gehärteten Schienen versehen sein. Schliesslich wird man die Scharbleche 15 an ihren der Reibung ausgesetzten Oberflächen vergüten. Solche Vergütungen können beispielsweise hochlegierte Stahlbleche sein, oder auch nur eine Oberflächenhärtung oder aber auch Beschichtungen in Form von Keramikplatten. Die entsprechenden Möglichkeiten müssen auf ihre Tauglichkeit überprüft werden.

Auf Grund der beschriebenen Konstruktion der Rührarme 12 und der Schaufeln 13 ist nunmehr das anzuwendende Verfahren deutlich erklärbar. Der gefüllte Fermenter 1 durchläuft eine Ruhephase, welche eine Zeit t₃ beträgt. Während dieser Ruhephase werden sich schwerere Feststoffe nach unten im Fermenter absenken. Es bildet sich während dieser Zeit somit eine Sedimentationsschicht am Boden des Fermentertanks 2. Diese Schicht soll in einem ersten Schritt aufgetrennt werden. Hierzu wird man die Welle 10 in jene Richtung drehen, dass die Rührarme 12 dank der Scharbleche 15 eine pflügende Wirkung ausüben. Diese Drehrichtung wird während einer Zeit t₁ beibehalten. Nach Ablauf der Zeit t₁ wird die Welle 10 angehalten und sogleich in der Gegenrichtung in Betrieb gesetzt. Nun wirken die Schaufeln 13 in ihrem eigentlichen Sinn, nämlich fördernd, und fördern folglich die abgesunkenen schweren Anteile im biogenen Abfall 9 nach oben. Die Dauer der einzelnen Phasen, in denen die Welle in der einen oder in der anderen Richtung dreht, beziehungsweise in der die Welle still steht, ist selbstverständlich abhängig von der Zusammensetzung der biogenen Abfälle, von der Grösse des Fermenters 1 und anderen Parametern. Prinzipiell ist dies jedoch für den Betreiber problemlos steuerbar, da er ohnehin verschiedenen Parameter überwacht, die die entsprechenden Einflussgrössen bilden. So wird prinzipiell der pH-Wert festgestellt, die Methangasbildung überwacht, der Feuchtigkeitsgehalt der biogenen Abfälle gesteuert und die Temperatur überwacht. All diese Grössen beeinflussen die Zeiten, in denen die Welle dreht beziehungsweise still steht.

Der Widerstand der in der pflügenden Drehrichtung zu überwinden ist, ist geringer als in der fördernden Drehrichtung. Rechnerisch beträgt das aufzubringende Drehmoment rund 20 % weniger in der pflügenden Drehrichtung als in der fördernden Drehrichtung. Bei grösseren Fermentern wird folglich nach der Stillstandszeit das Rührwerk immer erst in der pflügenden Richtung betrieben. Bei kleineren Fermentern ist jedoch diese Reduktion des Drehmoments weniger wesentlich und daher kann in diesem Fall die Reihenfolge der Drehrichtungen beliebig sein, auch wenn hier die erst beschriebene Reihenfolge der Drehrichtungen ebenfalls bevorzugt wird.

Erfahrungsgemäss wird man die Zeit t₂, in der das Rührwerk in Förderrichtung dreht in einem Bereich zwischen 3 und 10 Minuten wählen. Die Zeit, in der das Rührwerk in pflügender Richtung dreht, liegt vorzugsweise in einem Bereich von 1 bis 5 Minuten. Die Stillstandszeit sollte mindestens in etwa der Summe der Zeiten, in denen das Rührwerk in fördernder und in pflügender Richtung läuft, entsprechen. Die Ruhezeit kann aber durchaus auch länger sein, wenn der Trockensubstanzanteil der zu vergärenden biogenen Abfälle besonders hoch ist. Bevorzugterweise beträgt die Stillstandszeit zwischen 5 und 15 Minuten, doch kann sie auch wesentlich höher, beispielsweise bis zu einer Zeit von 30 bis 60 Minuten betragen. Die Stillstandszeit wird man optimieren, um einerseits zwar eine genügende Durchmischung zu haben, andererseits jedoch den Energieverbrauch möglichst tief zu halten.

Auch die Rotationsgeschwindigkeit der Welle ist im Prinzip frei wählbar. Wie aus dem bisherigen Verfahren bekannt, sind Rotationsgeschwindigkeiten der Welle zwischen 0,5 und 3 U/min. realistische Grössenordnungen. Höhere Rotationsgeschwindigkeiten führen zu einem unnötigen Energieverbrauch und einer zu starken Durchmischung, während tiefere Rotationsgeschwindigkeiten eine genügende Durchmischung nicht mehr garantieren.

### Bezugszeichenliste:

- 1: liegender Fermenter
- 2: Tank
- 3: Einlass
- 4: einlassseitige Stirnwand
- 5: Auslass
- 6: auslassseitige Stirnwand
- 7: Wellenlager
- 8: Wellenzapfen
- 9: biogene Abfälle
- 10: Welle
- 11: Wellenkörper
- 12: Rührarme
- 13: Schaufeln
- 14: Flanschplatten
- 15: Scharbleche
- 16: gemeinsame Kante
- 16': V-förmige Schiene
- 17: Oberflächenvergütung
- 18: Schaufelbleche
- 19: Kratzblech
- 20: Doppel-T-Träger
- 21: Längsflanschen
- 22: Verbindungssteg
- 23: Verlängerung des Verbindungssteges
- 30: Bugverlängerung
- 31: Bugblech

## Patentansprüche

1. Verfahren zum Betrieb eines pfropfstrombetriebenen Fermenters (1) zur anaeroben Fermentation biogener Abfälle (9) mit einem Einlass (3) und einem Auslass (4) und einem Rührwerk, welches aus einer den Fermenter in seiner Längsrichtung durchquerenden Welle (10) besteht, an der eine Vielzahl von Rührarmen (12) angeordnet sind, wobei die Welle (10) im Bereich der Fermenterenden (4,6) gelagert ist, **dadurch gekennzeichnet, dass** das Rührwerk Schaufeln (13) aus zwei V-förmig angeordneten Scharblechen (15) aufweist und in folgenden Schritten betrieben wird:
Schritt a) während einer Zeit t₁ dreht das Rührwerk in einer Richtung, wobei mit den V-förmig angeordneten Scharblechen (15) eine pflügende Wirkung erzielt wird;
Schritt b) während einer Zeit t₂ dreht das Rührwerk in der gegenläufigen Richtung, wobei die Schaufeln (13) eine fördernde Wirkung erzielen.
Schritt c) während einer Zeit t₃ steht das Rührwerk still
womit ein Zyklus abgeschlossen ist und nach Ablauf der Zeit t₃ der Zyklus sich wiederholt.

2. Verfahren nach Anspruch 1, **dadurch gekennzeichnet, dass** die Reihenfolge der Schritte a), b) c) ist und mit Schritt a) oder c) beginnt.

3. Verfahren nach Anspruch 1, **dadurch gekennzeichnet, dass** die Reihenfolge der Schritte b), a) c) ist und mit dem Schritt b) oder c) beginnt.

4. Verfahren nach Anspruch 1, **dadurch gekennzeichnet, dass** die Stillstandszeit t₃ mindestens gleich lang wie die Summe der Zeiten t₁ und t₂, in denen das Rührwerk in die eine Richtung und in die Gegenrichtung dreht, ist.

5. Verfahren nach Anspruch 1, **dadurch gekennzeichnet, dass** die Zeit t₂, in der das Rührwerk in der einen Richtung dreht, zwischen drei bis zehn Minuten beträgt und die Zeit t₁, in der das Rührwerk in die Gegenrichtung dreht, eine und fünf Minuten beträgt.

6. Verfahren nach Anspruch 1, **dadurch gekennzeichnet, dass** die Rotationsgeschwindigkeit der Welle zwischen 0,5 und 3 Umdrehungen pro Minute beträgt.

7. Vorrichtung zur Ausübung des Verfahrens nach Anspruch 1, umfassend einen liegenden Fermenter (1) bestehend aus einem Tank (2) und einer diesen in seiner Längsrichtung durchsetzenden getriebenen Welle (10) mit einer Vielzahl von daran angeordneten Rührarmen (12) mit Schaufeln (13), **dadurch gekennzeichnet, dass** die Schaufeln (13) aus zwei V-förmig angeordneten Scharblechen (15) gebildet sind, die in einer Drehrichtung als Schaufel und in der anderen Drehrichtung des Rührwerks als Pflugschar wirken.

8. Vorrichtung nach Anspruch 7, **dadurch gekennzeichnet, dass** die Schaufeln zwischen den beiden V-förmig angeordneten Scharblechen (15) eine in der Winkelhalbierenden verlaufende Versteifung (23) aufweisen und ein Schaufelblech (18) zwischen den beiden Scharblechen eingeschweisst ist, das senkrecht auf die Versteifung (23) sich abstützend angeordnet ist.

9. Vorrichtung nach Anspruch 7, **dadurch gekennzeichnet, dass** die Scharbleche (15) auf der pflügend wirkenden Seite vergütet (17) sind.

10. Vorrichtung nach Anspruch 9, **dadurch gekennzeichnet, dass** die Vergütung (17) eine Oberflächenhärtung ist.

11. Vorrichtung nach Anspruch 9, **dadurch gekennzeichnet, dass** die Vergütung (17) durch eine Hartmetallblechauflage gebildet ist.

12. Vorrichtung nach Anspruch 7, **dadurch gekennzeichnet, dass** die gemeinsame Vorderkante (16) der beiden Scharbleche (15) durch eine Hartmetallschiene (16') mit V-Profilquerschnitt geschützt ist.

13. Vorrichtung nach Anspruch 8, **dadurch gekennzeichnet, dass** die Rührarme aus Doppel-T-Trägern (20) gebildet sind, deren Ende im Bereich der Scharbleche in Einfach-T-Träger abgeändert sind, wobei der verbindende Steg (22) der Doppel-T-Träger in der Winkelhalbierenden zwischen den beiden Scharblechen stehen bleibt und mit einer Verlängerung (23) zur Versteifung gestaltet ist.

14. Vorrichtung nach Anspruch 8, **dadurch gekennzeichnet, dass** die Pflugscharbleche (15) über die Höhe der Schaufeln (13) hinweg in Richtung der Welle auf der Breite der Rührarme (12) als Bugverlängerungen (30) gebildet sind.

15. Vorrichtung nach Anspruch 14, **dadurch gekennzeichnet, dass** die Bugverlängerungen aus einem getrennten, V-förmig gestalteten Blech als Bugblech gebildet sind.

## Claims

1. Method for operating a horizontal plug-flow fermenter (1) for the anaerobic fermentation of biogenic waste (9) with an inlet (3) and an outlet (4) and an agitator which consists of a shaft (10) passing through the fermenter in its longitudinal direction, on which a plurality of agitator arms (12) are arranged, the shaft (10) being mounted in the region of the fermenter ends (4, 6), **characterised in that** the agitator comprises paddles (13) made up of two shares (15) arranged in a V-shaped manner and is operated in the following steps:
Step a) during a time t₁ the agitator rotates in one direction, a ploughing effect being achieved by the shares (15) arranged in a V-shaped manner;
Step b) during a time t₂ the agitator rotates in the opposite direction, the paddles (13) achieving a conveying effect.
Step c) during a time t₃, the agitator is stationary,
whereby a cycle is completed and after the passing of the time t₃, the cycle is repeated.

2. Method according to Claim 1, **characterised in that** the sequence of the steps is a), b), c) and begins with step a) or c).

3. Method according to Claim 1, **characterised in that** the sequence of the steps is b), a), c) and begins with the step b) or c).

4. Method according to Claim 1, **characterised in that** the stationary time t₃ is at least as long as the sum of the times t₁ and t₂, in which the agitator rotates in the one direction and in the opposite direction.

5. Method according to Claim 1, **characterised in that** the time t₂, in which the agitator rotates in the one direction is between three to ten minutes and the time t₁ in which the agitator rotates in the opposite direction is one and five minutes.

6. Method according to Claim 1, **characterised in that** the rotational speed of the shaft is between 0.5 and 3 revolutions per minute.

7. Device for carrying out the method according to Claim 1, comprising a horizontal fermenter (1) consisting of a tank (2) and a driven shaft (10) penetrating said tank in its longitudinal direction, comprising a plurality of agitator arms (12) arranged thereon with paddles (13), **characterised in that** the paddles (13) are formed from two shares (15) arranged in a V-shaped manner which act in one rotational direction as paddles and in the other rotational direction of the agitator as plough shares.

8. Device according to Claim 7, **characterised in that** the paddles between the two shares (15) arranged in a V-shaped manner comprise a reinforcement (23) extending in the angle bisector and a paddle sheet (18) is welded between the two shares, which is arranged perpendicular to the reinforcement (23) in a supporting manner.

9. Device according to Claim 7, **characterised in that** the shares (15) are hardened and tempered (17) on the side effecting the ploughing.

10. Device according to Claim 9, **characterised in that** the hardening and tempering (17) is a surface hardening.

11. Device according to Claim 9, **characterised in that** the hardening and tempering (17) is created by a hard sheet metal support.

12. Device according to Claim 7, **characterised in that** the common front edge (16) of the two shares (15) is protected by a hard metal rail (16') with a V-profile cross section.

13. Device according to Claim 8, **characterised in that** the agitator arms are formed from double T-girders (20), the ends thereof being altered in the region of the shares in the single T-girder, the connecting rib (22) of the double T-girders remaining fixed in the angle bisector between the two shares and being designed with an extension (23) for reinforcement.

14. Device according to Claim 8, **characterised in that** the plough shares (15) are formed above the level of the paddles (13) in the direction of the shaft along the width of the agitator arms (12) as front extensions (30).

15. Device according to Claim 14, **characterised in that** the front extensions are formed from a separate V-shaped metal plate designed as a front plate.

## Revendications

1. Procédé pour le fonctionnement d'un fermenteur (1) alimenté par un courant de greffage pour la fermentation anaérobie de déchets (9) biogènes comprenant une entrée (3) et une sortie (4) et un agitateur, lequel est constitué d'un arbre (10) traversant le fermenteur dans sa direction longitudinale, sur lequel sont disposés une pluralité de bras d'agitateur (12), l'arbre (10) étant monté dans la zone des extrémités de fermenteur (4, 6), **caractérisé en ce que** l'agitateur présente des ailettes (13) constituées de deux tôles de soc (15) disposées en V et est exploité avec les étapes suivantes:
Etape a) pendant un temps t₁, l'agitateur tourne dans une direction, un effet de labourage étant obtenu avec les tôles de soc (15) disposées en V,
Etape b) pendant un temps t₂, l'agitateur tourne dans le sens contraire, les ailettes (13) permettant d'obtenir un effet d'avancement,
Etape c) pendant un temps t₃, l'agitateur est immobilisé,
un cycle étant terminé et le cycle se répétant après l'écoulement de la durée t₃.

2. Procédé selon la revendication 1, **caractérisé en ce que** l'ordre de succession des étapes est a), b), c) et commence avec a) ou c).

3. Procédé selon la revendication 1, **caractérisé en ce que** l'ordre des étapes est b), a) c) et commence avec l'étape b) ou c).

4. Procédé selon la revendication 1, **caractérisé en ce que** le temps d'arrêt t₃ est au moins aussi long que la somme des temps t₁ et t₂, au cours desquels l'agitateur tourne dans une direction et dans la direction inverse.

5. Procédé selon la revendication 1, **caractérisé en ce que** le temps t₂, lors duquel l'agitateur tourne dans une direction, est compris entre trois et dix minutes et le temps t₁, lors duquel l'agitateur tourne dans la direction opposée, est de une et cinq minutes.

6. Procédé selon la revendication 1, **caractérisé en ce que** la vitesse de rotation de l'arbre se situe entre 0,5 et 3 rotations par minute.

7. Dispositif pour mettre en oeuvre le procédé selon la revendication 1, comprenant un fermenteur (1) horizontal comportant un réservoir (2) et un arbre (10) entraîné et traversant ce réservoir dans sa direction longitudinale avec une pluralité de bras d'agitateur (12) disposés dessus avec des ailettes (13), **caractérisé en ce que** les ailettes (13) sont formées de deux tôles de soc (15) disposées en V, qui agissent dans un sens de rotation comme ailette et dans l'autre direction de l'agitateur comme soc de charrue.

8. Dispositif selon la revendication 7, **caractérisé en ce que** les ailettes présentent entre les deux tôles de soc (15) disposées en V un renfort (23) agencé dans la bissectrice et une tôle d'ailette (18) est soudée entre les deux tôles de soc, laquelle est disposée verticalement en s'appuyant sur le renfort (23).

9. Dispositif selon la revendication 7, **caractérisé en ce que** les tôles de soc (15) sont traitées sur le côté ayant un effet de labourage.

10. Dispositif selon la revendication 9, **caractérisé en ce que** le traitement (17) est un durcissement superficiel.

11. Dispositif selon la revendication 9, **caractérisé en ce que** le traitement (17) est formé par un support de tôle en métal dur.

12. Dispositif selon la revendication 7, **caractérisé en ce que** l'arête avant (16) commune des deux tôles de soc (15) est protégée par un rail en métal dur (16') avec une section de profilé en V.

13. Dispositif selon la revendication 8, **caractérisé en ce que** les bras d'agitateur sont formés de supports en double T (20), dont les extrémités sont modifiées dans la zone des tôles de soc en supports à simple T, la nervure (22) de liaison des supports en double T restant dans la bissectrice entre les deux tôles de soc et étant conçue avec un prolongement (23) pour le renfort.

14. Dispositif selon la revendication 8, **caractérisé en ce que** les tôles de soc de charrue (15) sont formées à la hauteur des ailettes (13) en direction de l'arbre sur la largeur des bras d'agitateur (12) sous forme de prolongements avant (30).

15. Dispositif selon la revendication 14, **caractérisé en ce que** les prolongements avant sont formés à base d'une tôle séparée et conçue en V comme tôle avant.
